(19) Europäisches Patentamt
European Patent Office
Office européen des brevets

(11) EP 3 322 687 B1

(12) **EUROPEAN PATENT SPECIFICATION**

(45) Date of publication and mention of the grant of the patent:
**26.02.2020 Bulletin 2020/09**

(21) Application number: **16721511.0**

(22) Date of filing: **04.03.2016**

(51) Int Cl.:
***C07C 49/755*** (2006.01) ***C07D 317/54*** (2006.01)
***A61K 31/122*** (2006.01) ***A61K 31/36*** (2006.01)
***A61P 35/00*** (2006.01)

(86) International application number:
**PCT/IN2016/050075**

(87) International publication number:
**WO 2017/009860 (19.01.2017 Gazette 2017/03)**

(54) **2-BENZYL-INDANONE COMPOUNDS AS ANTICANCER AGENT AND A PROCESS FOR PREPARATION THEREOF**

2-BENZYL-INDANON-VERBINDUNGEN ALS ANTIKREBSMITTEL UND VERFAHREN ZUR HERSTELLUNG DAVON

COMPOSÉS 2-BENZYL-INDANONE EN TANT QU'AGENTS ANTICANCÉREUX ET PROCÉDÉ DE PRÉPARATION DE CEUX-CI

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **13.07.2015 IN 2105DE2015**

(43) Date of publication of application:
**23.05.2018 Bulletin 2018/21**

(73) Proprietor: **Council of Scientific and Industrial Research**
**New Delhi 110001 (IN)**

(72) Inventors:
- **NEGI, Arvind Singh**
  **Lucknow 226015 (IN)**
- **SINGH, Aastha**
  **Lucknow 226015 (IN)**
- **LUQMAN, Suaib**
  **Lucknow 226015 (IN)**
- **CHANDA, Debabrata**
  **Lucknow 226015 (IN)**
- **MONDHE, Dilip Manikrao**
  **Jammu 180001 (IN)**
- **SAXENA, Ajit Kumar**
  **Jammu 180001 (IN)**
- **FATIMA, Kaneez**
  **Lucknow 226015 (IN)**
- **SINGH, Arjun**
  **Lucknow 226015 (IN)**

(74) Representative: **AWA Sweden AB**
**P.O. Box 5117**
**200 71 Malmö (SE)**

(56) References cited:
**FR-A1- 2 838 432 US-B2- 8 633 242**

- **A.P. PRAKASHAM, ET AL.: "Synthesis and anticancer activity of 2-benzylidene indanones through inhibiting tubulin polymerisation", BIOORGANIC & MEDICINAL CHEMISTRY, vol. 20, no. 9, 14 March 2012 (2012-03-14) , pages 3049-3057, XP028412834, Elsevier Science Publishers, Oxford, GB ISSN: 0968-0896, DOI: 10.1016/j.bmc.2012.02.057**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).



Printed by Jouve, 75001 PARIS (FR)

## Description

### FIELD OF THE INVENTION

The present invention relates to 2-benzyl-indanone compounds as anticancer agents and the process of preparing the same. The invention particularly relates to the gallic acid based new molecules i.e. 2-benzyl indanones represented by general structure 1, possessing anticancer activity against human cancer cell lines. More particularly, the invention relates to the potent anticancer and tubulin polymerisation inhibition activity of a new benzyl-indanone (2) synthesized from gallic acid. This invention also provides a new process for the preparation of the said molecules from a naturally occurring compound i. e. gallic acid.

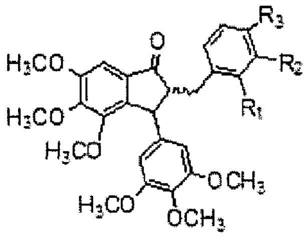

**1: General structure**

2: R1=H, R2-R3=O-CH2-O;
4: R1=R2=H, R3=F; 5: R1=R3=OCH3, R2=H

### BACKGROUND AND PRIOR ART OF THE INVENTION

Carcinogenesis is a process by which normal cells are transformed to cancer cells. DNA damage is considered as the primary cause of cancer. Cancer is the second largest killer accounting 8.2 million deaths in 2012. The morbidity and mortality of cancer is so high that it has become a burden to the society. Deaths from cancer worldwide are projected to continue rising, with an estimated over 11 million deaths in 2030. Chemotherapy, surgery and radiation therapy are some of the approaches to tackle this disease. But, there is no up to the mark cancer treatment available. In chemotherapy mostly cytotoxic drugs like paclitaxel, docetaxel, vincristine, vinblastine, cisplatin, 5-fluorouracil etc. are used to treat cancer. But, these drugs are not selective as they also kill the healthy cells. Due to which there is high level of toxicity associated with these cancer drugs. For hormone dependent breast cancer, hormone therapy is done. Tamoxifen, an antiestrogenic drug is used, which is only antiproliferative in nature. So, adjuvant therapy is used where surgical removal of tumour is done and tamoxifen is given continuously to the patient. But, use of tamoxifen for longer period may induce ovarian cancer and in some cases resistance is also acquired by the tumour cells. Drug resistance is another problem associated with these drugs due to elongated treatment. In drug resistance, the use of combination therapy, which is the administration of several drugs with different and complimentary mechanisms of action, is regarded as the more effective approach. But, the side effects are also additive due to multiple therapies. Therefore, the object of today is to overcome the shortcomings of the present cancer chemotherapy with an anti-tumour drug with a new mechanism of action, capable of discriminating tumour cells from normal proliferate cells and exhibiting selectivity against cancer.

Gallic acid (**2**), a plant phenolic acid, is present in almost all woody perennials. It is present as hydrolysable tannins. Being a small molecule with scope to modify and natural abundance, it has been a building block of choice for several synthetic bioactive lead molecules. Gallic acid and its compounds have exhibited potential pharmacological activities [Locatelli etal., Eur. J. Med. Chem., 2013, 60: 233-239; Pettit etal., J. Nat. Prod. 2000, 63(7), 969-974]

Indanones are very important class of naturally occurring bioactive compounds [Syrchina and Semenov, Chem, Nat. Compds. 1982, 18: 1-11]. These compounds have mainly been explored as anticancer agents [Lawrence etal., Tetrahedron Lett. 2006, 47, 1637]. Indanocine and its analogues are being developed to combat drug resistance malignancies [Leoni etal., J. Natl. Cancer Inst. 2000, 92, 217]. Indanocine is a synthetic indanone molecule presently an investigational cancer drug by NCI, USA. It is a microtubule destabilizer [Biochem. Pharmacol. 2012; 83:1495-506]. It occupies colchicine binding site of tubulin [Das etal, 2009, Biochemistry, 48:1628-1635]. Kamimura D. et.al. [Jpn. Kokai Tokkyo Koho (**1996**), JP 08198798 A 19960806] isolated an antitumour indanone compounds i.e. 5-bromo-4,7-dihydroxyindan-1-one from animal sponge inhibiting proliferation of mouse lymphatic leukemia cells. Brendel K. et. al. [Hung. Pat. Appl. (2000), HU 9903620 A2 20000228] developed a pharmaceutical composition of some benzylidene indenyl formamides for the use as anticancer drugs. Bansal et al. [PCT Int. Appl. (2007), WO 2007031833 A2 20070322] prepared some indan-1-one compounds as aromatase inhibitors. Aromatase inhibitors are used for breast cancer and ovarian cancer. Aromatase inhibitors block biosynthesis of estrogens.

Previously, 2-benzylidene indanones have been reported as potential anticancer agents. The anticancer activity of these compounds was through tubulin polymerisation inhibition [Negi et al., US8633242B2, January 21, 2014].
Synthesis and anticancer activity of 2-benzylidene indanones through inhibiting tubulin polymerization has been reported by Prakasham et. al., Biorganic and Medicinal Chemistry, 2012, 20(9), 3049-3057 (XP028412834)

## OBJECTIVE OF THE INVENTION

The main object of the present invention is to provide 2-benzyl-indanone compounds as anticancer agents and the process of preparing the same.
Another object of the invention is to provide a new anticancer molecule i.e. 2-(3',4'-methylenedioxybenzyl)-3-(3",4",5"-trimethoxyphenyl)-indanone-1 represented by structure **2.**
It is also an object of the invention to provide a process of preparation of these biologically active molecules represented by structure **1** in good yields, from readily available starting material gallic acid (**6**).
Yet another object of the invention is to provide an effective amount of new compound **2** as anticancer agents.

## SUMMARY OF THE INVENTION

Accordingly the present invention provides a compound of general formula 1

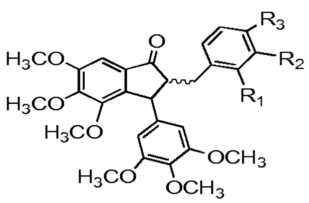

**1**

wherein R1, R2, and R3 are selected from a group consisting of H, Halogen, alkyl, alkoxy, alkylenedioxy, hydroxy, and aryl; wherein, R3 is not hydroxy when $R_1$ and $R_2$ are H.
In an embodiment of the invention wherein the representative compounds of formula 1 comprises;
2-(3',4'-methylenedioxybenzyl)-3-(3",4",5"-trimethoxyphenyl)-indanone-1;
2-(4'-fluorobenzyl)-3-(3",4",5"-trimethoxyphenyl)-indanone-1;
2-(2',4'-dimethoxybenzyl)-3-(3",4",5"-trimethoxyphenyl)-indanone-1.
In another embodiment of the invention wherein the structural formula of the compound comprising;

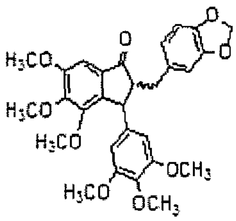

**2**

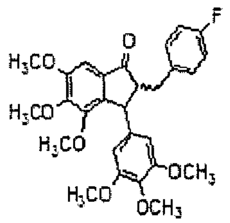

**4**

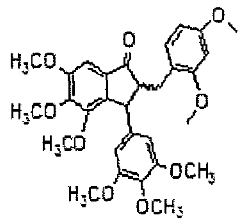

**5**

In yet another embodiment of the invention wherein the compound represented by formula **2** has tubulin polymerisation inhibition activity.
In a further embodiment of the invention wherein the compound is useful as an anticancer agent.
In one more embodiment of the invention wherein the compound represented by formula **2** exhibits cytotoxicity against various human cancer cell lines, $IC_{50}$ ranging from 1$\mu$M to 3 $\mu$M.
In still another embodiment of the invention wherein the compound of formula 2 shows no toxicity in *in-vivo* acute oral toxicity up to 300mg/kg.
In an embodiment of the invention wherein the compound of formula 2 exhibits *in-vivo* anticancer activity in Swiss-albino mice reduces Ehrlich ascite carcinoma by 67.64% and solid tumour by 42.77% at 20mg/kg dose.
Accordingly, the present invention also provides a process for the preparation of the compounds of general formula **1,** wherein the process step comprising;

a. adding a compound selected from a group consisting of 12, 14 and 15,

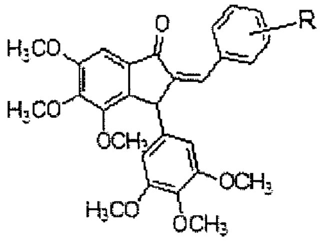

**12**: R= 3,4,-methylenedioxy;
**14**: 4-Fluoro; **15**: 2,4-Dimethoxy

in a solvent under stirring at a temperature 20-45°C for a period of time ranging between 5 to 10 min to get solution;
b. adding catalyst to the solution obtained from step (a) under hydrogen atmosphere followed by stirring at a temperature ranging between 20-45°C for a period of time 5 h to get reaction mixture;
c. filtering the reaction mixture obtained from step (b) followed by washing and drying to get pure product.
In an embodiment of the invention wherein the solvent used in step (a) is selected from the group consisting of methanol, dioxane, ethyl acetate and tetrahydrofuran.

In an embodiment of the invention wherein the catalyst used in step (b) is selected from the group consisting of Renay-Ni, RhCl (PPh3) and Pd-C.

In an embodiment of the invention wherein the amount of catalyst used in step (b) is in the range of 0.5 g to 1.0g per gram of substrate.

In an embodiment of the invention wherein the yield of the compound 1 in the range of 40-83%.

A pharmaceutical composition for the treatment of cancer wherein the composition comprises an effective amount of the compound of general formula I optionally along with the pharmaceutically acceptable excipients, diluents, additives.

In an embodiment of the invention wherein the diluents, additives used is selected from a group consisting of DMSO or Carboxymethylcellulose, gum acacia or cremophore EL,

A compound of general formula I for use in a method of treating cancer or any other disease related to tubulin disorders wherein the method comprises administering a therapeutically effective amount of one or more compounds of formula 1;

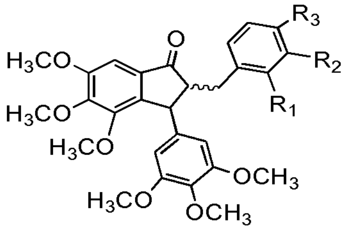

**1**

Where R1, R2, and R3 are selected from a group consisting of H, Halogen, alkyl, alkoxy, alkylenedioxy, hydroxy, and aryl; wherein, R3 is not hydroxy when $R_1$ and $R_2$ are H.

## BRIEF DESCRIPTION OF THE DRAWINGS

**Figure 1.** Effect of Compound **2** as a single acute oral dose at 5, 50, 300 and 1000 mg/kg on absolute and relative organ weight in Swiss albino mice (n=6, Non significant changes were found compared to control).
**Figure 2.** Effect of compound 2 as a single acute oral dose at 5, 50, 300 and 1000 mg/kg body weight on differential leucocytes counts in Swiss albino mice (n=6, Non significant changes were found compared to control).

## DETAILED DESCRIPTION OF THE INVENTION

The present invention provides 2-benzyl-indanone compounds as anticancer agent and the process of preparing the same. The invention also relates to four new molecules i.e. 2-(3',4'-methylenedioxybenzyl)-3-(3",4",5"-trimethoxyphenyl)-indanone-1 (**2**), 2-(4'-fluorobenzyl)-3-(3",4",5"-trimethoxyphenyl)-indanone-1 (**4**), 2-(2',4'-dimethoxybenzyl)-3-(3",4",5"-trimethoxyphenyl)-indanone-1 (**5**), represented by structural formula (**2, 4, 5**) as shown herein;

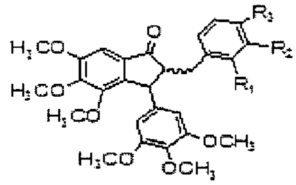

**1: General structure**

2: R1=H, R2-R3=O-CH2-O;
4: R1=R2=H, R3=F; 5: R1=R3=OCH3, R2=H

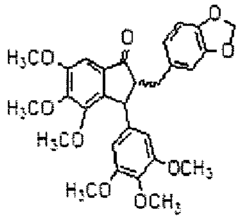

**2**

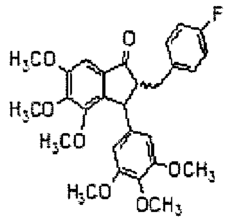

**4**

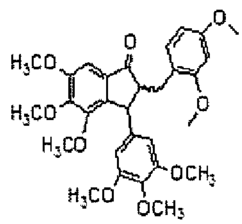

**5**

These compounds exhibit *in-vitro* anticancer activity against various human cancer cell lines. The compound **2** exhibits potential *in-vitro* and *in-vivo* anticancer activity. Its mode of action is ascertained through tubulin polymerisation inhibition activity. Tubulin polymerisation is an important step in cell cycle and any disruption leads to cell cycle arrest, which ultimately kills the cell. Compound **2** is found non-toxic and well tolerated by Swiss-albino mice in acute oral toxicity. The present invention provides a new molecule i.e. 2-(3,4-Methylenedioxybenzyl), 3-(3,4,5-trimethoxyphenyl), 4,5,6-trimethoxyindanone represented by structural formula (**2**) as shown herein;

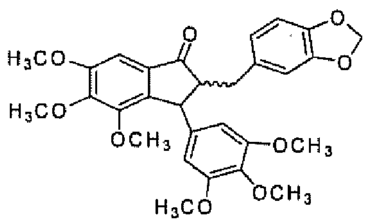

**2**

The compound exhibits anticancer and tubulin polymerisation inhibition activity.

**Synthesis:** The process for preparation of this novel compound comprises six synthetic steps of; a) Methylation of gallic acid (**6**) with a methylating agent to get a trimethoxy-methyl benzoate (7); (b) Reduction of ester to trimethoxybenzyl alcohol (**8**) with a suitable reducing agent; (c) Oxidation of benzyl alcohol to trimethoxybenzaldehyde (**9**) with a suitable oxidizing agent; (d) Condensation of trimethoxybenzaldehyde with a trimethoxyacetophenone in an alkaline solution to get a chalcone unit (**10**); (e) Conversion of chalcone unit to indanone moiety (**11**) by treating with a reagent; (f) Condensation of the indanone unit with a suitable aromatic aldehyde in alkaline solution to obtain benzylidene compound **12;** (g) Reduction of 2-benzylidene group to 2-benzyl to obtain final compound **2.**

Similarly, other members of this series (4-**5**) have also been synthesized by using different benzylidene compounds (**12**, **14, 15**) in step (g) under similar reaction conditions to get benzyl indanones having structural formula **3-5.**

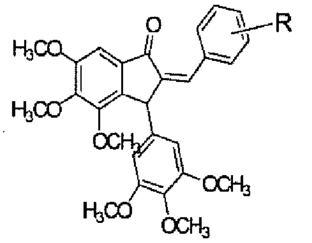

**12**: R= 3,4,-methylenedioxy; ~~**13**: 4-benzyloxy;~~
**14**: 4-Fluoro; **15**: 2,4-Dimethoxy

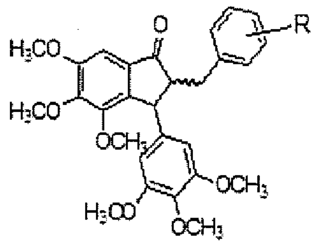

**2**: R= 3,4,-methylenedioxy;
**4**: 4-Fluoro; **5**: 2,4-Dimethoxy

2-benzo[1,3]dioxo-5-ylmethylene-4,5,6-trimethoxy-3-(3,4,5-trimethoxyphenyl)-indan-1-one (**12**) yields 2-(3",4"-methylenedioxybenzyl)-3-(3',4',5'-trimethoxyphenyl)-4,5,6-trimethoxy-indan-1-one (**2**); 2-(4"-fluorobenzylidene)-3-(3',4',5'-trimethoxyphenyl)-4,5,6-trimethoxy-indan-1-one] (**16**) yields 2-(4"-fluorobenzyl)-3-(3',4',5'-trimethoxyphenyl)-4,5,6-trimethoxy-indan-1-one] (**4**); while, 2-(2",4"-dimethoxybenzylidene)-3-(3',4',5'-trimethoxyphenyl)-4,5,6-trimethoxy-indan-1-one] (**13**) yields 2-(2",4"-dimethoxybenzyl)-3-(3',4',5'-trimethoxyphenyl)-4,5,6-trimethoxy-indan-1-one] (**5**). The details of examples 2, 4, 5 is given in the Table 1 below;

**Table 1:** Physical data of examples 2, 4, 5

| Compd. No. | Yield (%) | m.p. °C | Spectral Data |
|---|---|---|---|
| **2** | 83 | 121-123 | $^1$HNMR (300MHz, $CDCl_3$): δ 2.22 (m,2H, benzylic), 2.50 (m,1H, 2-CH), 3.29 (s,3H, OCH3), 3.54 (s, 6H, 2xOCH3), 3,71 (s, 3H, OCH3), 3.79 (s, 3H, OCH3), 3.83 (s, 3H, OCH3), 4.58 (d, 1H, 3-CH, J=6.9Hz), 5.88 (s, 4H, -O-CH2-O- and 2" & 6"-CH), 6.2 (d, 1H, 5'-CH, J=7.8Hz), 6.29 (s, 1H, 2'-CH), 6.61 (d, 1H, 6'-CH), 7.11 (s, 1H, 7-CH). 13C NMR (75MHz, CDCl3): δ 30.07, 32.24, 46.21, 55.30, 56.41, 56.65, 60.69, 61.32, 61.38, 100.77, 101.14, 108.16, 109.48, 121.76, 131.53, 134.04, 136.39, 137.33, 143.25, 147.73, 149.11, 150.47, 153.22, 155.29, 206.07. Electrospray Ionization Mass (ESIMS, MeOH): 523.3 $[M+H]^+$, 545.3 $[M+Na]^+$, 561.2 $[M+K]^+$ . Electrospray Ionisation-High resolution mass (ESI-HRMS): Calcd, 523.19686; found, 523.19589 for $C_{29}H_{31}O_9$ $[M+H]^+$. |
| **4** | 76 | oil | $^1$H NMR ($CDCl_3$, 300MHz): δ 2.56 (t, 1H), 3.23 (bs, 2H), 3.29 (s, 3H), 3.55 (s, 6H), 3.74 (s, 3H), 3.87 (s, 3H), 4.07 (bs, 1H), 5.76 (s, 2H), 6.81-7.19 (m, 5H); ESIMS (MeOH): 497 $[M+H]^+$, 519 $[M+Na]^+$, 535 $[M+K]^+$, |
| **5** | 73 | 118-119 | $^1$H NMR ($CDCl_3$, 300MHz): δ 2.20 (t, 1H), 3.30 (d, 2H), 3.54-3,00 (m, 24H, 8xOCH3), 4.49 (d, 1H), 5.78 (s, 2H), 6.24 (d, 1H), 6.34 (s, 1H), 6.46 (d, 1H), 7.11 (m, 1H); ESIMS (MeOH): 539 $[M+H]^+$, 561 $[M+Na]^+$, 577 $[M+K]^+$. |

**A. Synthesis:** The process for preparation of this novel compound comprises seven synthetic steps of; a) Methylation of gallic acid (**6**) with a methylating agent to get a trimethoxy methyl benzoate (7); (b) Reduction of ester to trimethoxybenzyl alcohol (**8**) with a suitable reducing agent; (c) Oxidation of benzyl alcohol to trimethoxybenzaldehyde (**9**) with a suitable oxidizing agent; (d) Condensation of trimethoxybenzaldehyde with trimethoxyacetophenone in an alkaline solution to get a chalcone unit (**10**); (e) Conversion of chalcone unit to indanone moiety (**11**) by treating with a reagent; (f) Condensation of the indanone unit with a suitable aromatic aldehyde in alkaline solution to obtain benzylidene-indanone **12,14,15** (Table 2), and (g) Finally, reduction of 2-benzylidene indanone (**12,14,15**) to 2-benzyl-indanone (**2,4,5**) by a suitable reducing agent (Table 1).

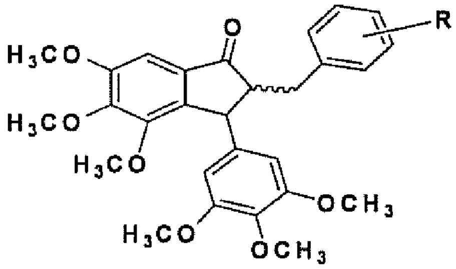

1

The methylating agent used in the step (a) may be selected from a group consisting of methyl iodide, diazomethane, and dimethyl sulphate. The reducing agent in the step (b) may be selected from the group consisting of sodium borohydride, lithium aluminium hydride, lithium borohydride, and diisobutyl aluminium hydride (DIBAL). The oxidizing agent in step (c) is Pyridinium chlorochromate or chromium trioxide-dipyridine complex or manganese dioxide or oxalyl chloride-dimethylsulphoxide. The base used in the step (d) is selected from the group consisting of sodium hydroxide, potassium hydroxide, sodium carbonate, potassium carbonate. The reagent used in the step (e) may be from a group of consisting trifluoroacetic acid, methanesulphonic acid and aluminium chloride. The base used in the step (f) may be selected from a group of bases consisting of potassium hydroxide, potassium carbonate, sodium hydroxide, sodium carbonate. The reducing agent in step (g) may selected from a group of reducing agents consisting sodium borohydride-trifluoroacetic acid, sodium borohydride-aluminium chloride, Wilkinson's catalyst and hydrogen-palladium-charcoal, hydrogen-Raney-nickel.

All the compounds **2, 4, 5** exhibit anticancer activity against various human cancer cell lines. The best compound of the series i.e. compound **2** exhibits anticancer activity against certain human cancer cell lines especially against MCF-7, HCT, THP-1 and A549 and tubulin polymerisation inhibition. It would have wide applicability in the pharmaceutical compositions. Without specifying any theory, the applicants state that compound **2** may be utilised in the pharmaceutical compositions for various cancer treatments or for any other disease related to tubulin disorders.

The invention, its embodiments and applications are described in detail in the examples given below which are provided to illustrate the invention and therefore should not be constructed to limit the scope of this invention.

The schematic diagram for the process of preparation is represented hereunder;

**Synthesis**

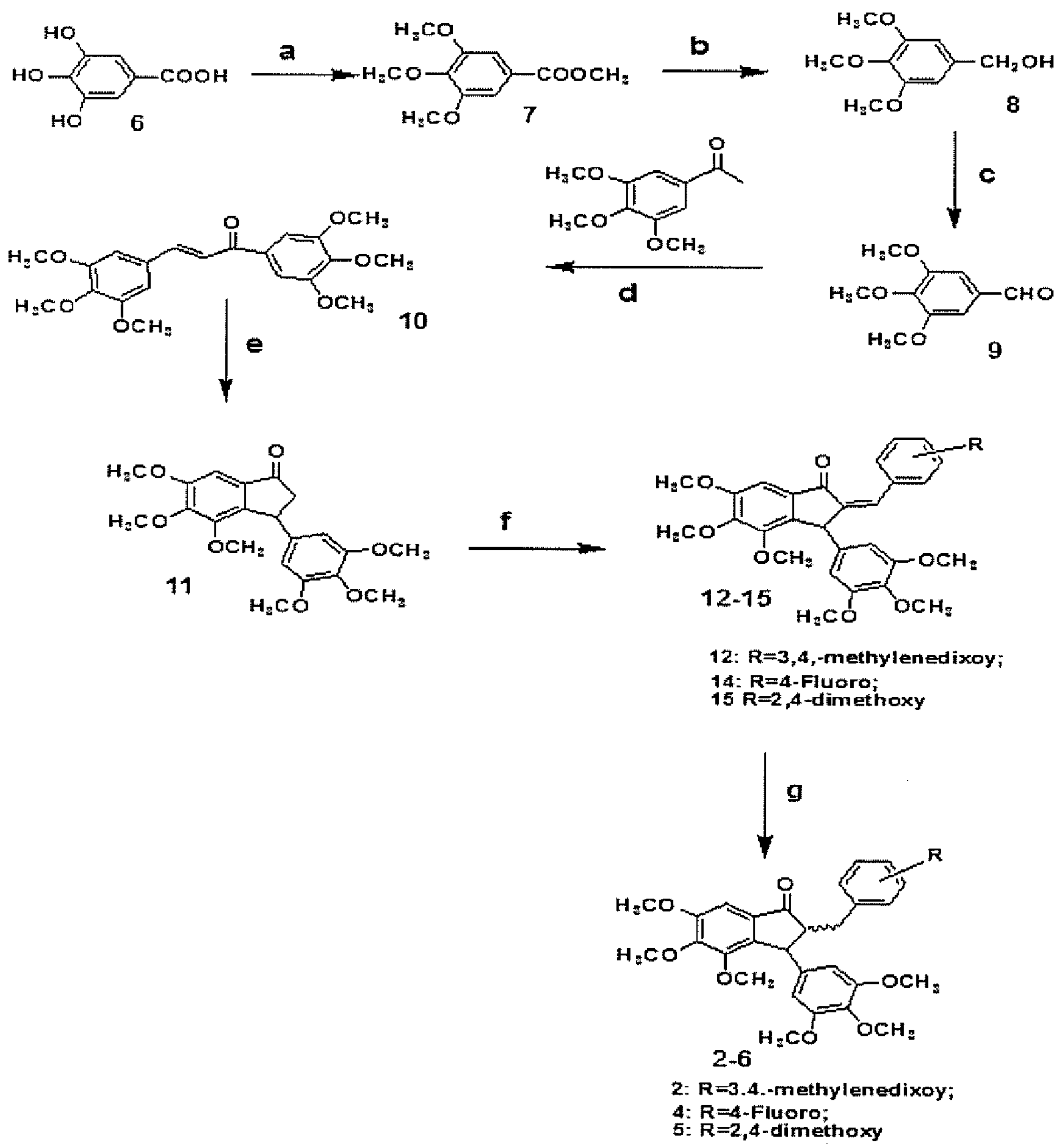


**Table 2:** Details of step for the synthesis of benzylidene compounds

| S. no. | Substrate | Aldehyde | Product | Yield (%) |
|---|---|---|---|---|
| 1. | **11** | 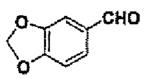 | **12** | 80% |
| 3. | **11** | 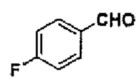 | **14** | 78% |
| 4. | **11** | 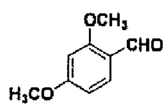 | **15** | 87% |

## EXAMPLES

Following examples are given by way of illustration and should not construe to limit the scope of the invention. The synthesis of representative compound 2-(3',4'-methylenedioxybenzyl)-3-(3",4",5"-trimethoxyphenyl)-indanone-1 (**2**) has been given.

### Example 1

**Step 1:** Preparation of 3,4,5-trimethoxybenzoic acid methyl ester (formula **7**)

A 50mL round bottom flask was charged with potassium carbonate (6g) and dry acetone (15mL) compound of formula **6** (2gm) and to it was added methyl iodide (4mL) and the reaction mixture was refluxed for 5 hours at 60°C with constant stirring. On completion, the reaction mixture was filtered and washed with acetone and distilled off to get compound of formula **7** (400mg).

**Step 2:** Preparation of 3,4,5-trimethoxybenzyl alcohol (formula **8**)

The compound having formula **7** (400mg) was stirred in tetrahydrofuran (10mL) with cooling. To this lithium borohydride (200mg) was added and further stirred for 3 hours. On completion, the reaction mixture was poured into water, acidified with dil. HCl and extracted with ethyl acetate. The organic layer was washed with water, dried over anhydrous sodium sulphate and evaporated to dryness to get the desired compound of formula **8** (340g).

**Step 3:** Preparation of 3,4,5-trimethoxybenzaldehyde (formula **9**)

The compound of formula **8** (340mg) was taken in dichloromethane (20mL). To this stirred solution dipyridine-chromium trioxide complex (500mg) was added and further stirred for 10 hours. On completion the reaction mixture was poured into water and extracted with ethyl acetate, Organic layer was washed with water, dried over anhydrous sodium sulphate and evaporated to dryness. The residue thus obtained was purified through silica gel column and eluted with hexane-ethyl acetate to get the desired compound of formula **9** (190mg).

**Step 4:** Preparation of 1,3-bis-(3,4,5-trimethoxyphenyl)-propenone (chalcone, formula 10)

To a stirred solution of 3,4,5-trimethoxybenzaldehyde (190mg, formula **9**) in 7% methanolic potassium carbonate (15mL), 3,4,5-trimethoxyacetophenone (200mg) was added. It was stirred for 3 hours. On completion, the reaction mixture was diluted with water, acidified with dil. HCl and extracted with ethyl acetate. The organic layer was washed with water, dried over anhydrous sodium sulphate and evaporated to get a residue. The residue was purified through silica gel column to get chalcone of formula **10** (240mg)

**Step 5:** Preparation of 4,5,6-trimethoxy-3-(3,4,5-trimethoxyphenyl)-indan-1-one (formula **11**):

1,3-bis-(3,4,5-trimethoxyphenyl)-propenone (chalcone, formula **10,** 240mg) was taken in toluene (10mL.). To reaction mixture methanesulphonic acid (1mL) was added and refluxed for 4 hours. On completion it was poured into cold water and extracted with ethyl acetate. The organic layer was washed with water, dried over anhydrous sodium sulphate and evaporated to get a residue. The residue was purified through silica gel column to get 4,5,6-trimethoxy-3-(3,4,5-trimethoxyphenyl)-indan-1-one (formula **11,** 32mg).

**Step 6:** Preparation of 2-benzo[1,3]dioxo-5-ylmethylene-4,5,6-trimethoxy-3-(3,4,5-trimethoxyphenyl)-indan-1-one (formula **12**)

4,5,6-trimethoxy-3-(3,4,5-trimethoxyphenyl)-indan-1-one (formula **11,** 32mg) was taken in 10% methanolic potassium hydroxide (15mL), 3,4-methylenedioxybenzaldehyde (20mg) was added. It was stirred for 3 hours. On completion, the reaction mixture was diluted with water, acidified with dil. HCl and extracted with ethyl acetate. The organic layer was washed with water, dried over anhydrous sodium sulphate and evaporated to get a residue. The residue was purified through silica gel column to get 2-benzo[1,3]dioxo-5-ylmethylene-4,5,6-trimethoxy-3-(3,4,5-trimethoxyphenyl)-indan-1-one (formula **12,** 16mg)

**Step 7:** Preparation of 2-(3',4'-methylenedioxybenzyl)-3-(3",4",5"-trimethoxyphenyl)-indanone-1 (**2**)

In a round bottom flask 2-benzo[1,3]dioxo-5-ylmethylene-4,5,6-trimethoxy-3-(3,4,5-trimethoxyphenyl)-indan-1-one (**12**, 200mg) was taken in MeOH (10mL). To this stirred solution, Raney-Ni (200mg) was added and hydrogen gas (200mL) was bubbled. The reaction was stirred for 2h at 30°C. On completion, the reaction mixture was filtered through celite bed, washed with methanol and evaporated *in-vacuo.* The residue was taken in ethyl acetate and washed with water, Organic layer was dried over anhydrous sodium sulphate and evaporated *in vacuo.* Compound 2 was obtained as light cream solid (140mg). Similarly, other compounds 4, **5** can also be prepared from compounds 14,**15** using similar reaction conditions.

**Example 2**

**Step 1:** Preparation of 3,4,5-trimethoxybenzoic acid methyl ester (formula 7)

In a 250mL round bottom flask 3g gallic acid was stirred at 10°C in 20% aqueous potassium hydroxide solution (100mL). To this dimethyl sulphate (4mL, 5.34g) was added drop-wise. The reaction mixture was stirred for one hour and the refluxed for 3 hours at 100°C. On completion, the reaction mixture was extracted with ethyl acetate, washed with water, dried over anhydrous sodium sulphate and distilled off to get a residue. It was recrystallised to get compound of formula 7 (1.2g).

**Step 2:** Preparation of 3,4,5-trimethoxybenzyl alcohol (formula **8**)

The compound having formula **7** (1.9 g) was dissolved in tetrahydrofuran (30mL). To this lithium-aluminium-hydride (1.16g) was added and refluxed for 1 hour. On completion, the reagent was decomposed by adding ethyl acetate, poured into water, acidified with dil. HCl and extracted with ethyl acetate. The organic layer was washed with water, dried over anhydrous sodium sulphate and evaporated to dryness to get a residue which on purification through silica gel column yielded the desired compound of formula **8** (1.06g).

**Step 3:** Preparation of 3,4,5-trimethoxybenzaldehyde (formula **9**)

The compound of formula **8** (1.0g) was taken in dichloromethane (25mL). To this stirred solution Pyridinium chlorochromate (1.38g) was added and further stirred at 30°C for one hour. On completion, the solvent was evaporated and the residue was poured into water and extracted with ethyl acetate. Organic layer was washed with water, dried over anhydrous sodium sulphate and evaporated to dryness. The residue thus obtained was purified through silica gel column to get the desired compound of formula **9** (875mg).

**Step 4:** Preparation of 1,3-bis-(3,4,5-trimethoxyphenyl)-propenone (chalcone, formula **10**)

To a stirred solution of 3,4,5-trimethoxyacetophenone (2.1g) in 7% methanolic-potassium hydroxide (15mL), 3,4,5-trimethoxybenzaldehyde (2.06g, formula **9**) was added. It was stirred at 30°C for 2 hour, The desired chalcone of formula **10** (3.8g) was obtained after usual workup and processed as such for next step.

**Step 5:** Preparation of 4,5,6-trimethoxy-3-(3,4,5-trimethoxyphenyl)-indan-1-one (formula **11**):

1,3-bis-(3,4,5-trimethoxyphenyl)-propenone (chalcone, formula **10,** 1g) was taken in trifluoroacetic acid (1mL) and heated at 140°C for 8 hours. On completion it was poured into cold water and extracted with ethyl acetate. The organic layer was washed with water, dried over anhydrous sodium sulphate and evaporated to get a residue. The residue was purified through silica gel column to get 4,5,6-trimethoxy-3-(3,4,5-trimethoxyphenyl)-indan-1-one (formula **11,** 160mg),

**Step 6:** Preparation of 2-benzo[1,3]dioxo-5-ylmethylene-4,5,6-trimethoxy-3-(3,4,5-trimethoxyphenyl)-indan-1-one (formula **12**)

4,5,6-trimethoxy-3-(3,4,5-trimethoxyphenyl)-indan-1-one (formula **7,** 500mg) was taken in 3% methanolic potassium hydroxide (10mL). To this stirred solution 3,4-methylenedioxybenzaldehyde (210mg) was added and further stirred at 30°C for 3 hours. On completion, the reaction mixture was diluted with water, acidified with dil. HCl and extracted with ethyl acetate. The organic layer was washed with water, dried over anhydrous sodium sulphate and evaporated to get a residue. The residue was purified through silica gel column to get 2-benzo[1,3]dioxo-5-ylmethylene-4,5,6-trimethoxy-3-(3,4,5-trimethoxyphenyl)-indan-1-one (formula **12,** 600mg)

**Step 7:** Preparation of 2-(3',4'-methylenedioxybenzyl)-3-(3",4",5"-trimethoxyphenyl)-indanone-1 (**2**)

In a round bottom flask 2-benzo[1,3]dioxo-5-ylmethylene-4,5,6-trimethoxy-3-(3,4,5-trimethoxyphenyl)-indan-1-one (**12**, 200mg) was taken in dry THF (10 mL). To this stirred solution, 10% Pd-C (200mg) was added and hydrogen gas (400mL) was supplied through a balloon. The reaction was stirred for 2h at 30°C. On completion, the reaction mixture was filtered through celite bed (or filter paper), washed with methanol and evaporated in vacuo. The residue was taken in ethyl acetate and washed with water. Organic layer was dried over anhydrous sodium sulphate and evaporated *in vacuo.* Compound **2** was obtained as light cream solid (170mg). Similarly, other compounds 4,**5** can also be prepared from compounds 14,**15** using similar reaction conditions.

**B. *In vitro* anticancer (cytotoxicity) activity against human cancer cell lines**

The benzyl compounds were evaluated by Sulforhodamine B Assay as per reported method [Skehan etal., J. Natl. Cancer Inst.1990, 82, 1107]. The human cancer cell lines procured from National Cancer Institute, Frederick, U. S. A. were used in present study. Cells were grown in tissue culture flasks in complete growth medium (RPMI-1640 medium with 2 mM glutamine, pH 7.4 supplemented with 10% fetal bovine serum, 100 μg/ml streptomycin and 100 units/ml penicillin) in a carbon dioxide incubator (37°C, 5% $CO_2$, 90% RH). The cells at subconfluent stage were harvested from the flask by treatment with trypsin (0.05% in PBS (pH 7.4) containing 0.02% EDTA). Cells with viability of more than 98%, as determined by trypan blue exclusion, were used for determination of cytotoxicity. The cell suspension required concentration of cells was prepared in complete growth medium. Stock solution of $2x10^{-2}$ M of compounds were prepared in DMSO and further dilution was carried out complete growth medium containing 50μg/ml of gentamycin to obtained working test solution of required concentration. *In vitro* cytotoxicity against various human cancer cell lines was determined using 96-well cell culture plates. The 100μl of cell suspension was added to each well of the 96-well cell culture plates. The cells were allowed to grow in $CO_2$ incubator (37°C, 5% $CO_2$, 90% RH) for 24 hours. The test materials in complete growth medium (100 μl) were added after 24 hours incubation to the wells containing cell suspension. The plates were further incubated for 48 hours (37°C in an atmosphere of 5% $CO_2$ and 90% relative humidity) in a carbon dioxide incubator after addition of test material and then the cell growth was stopped by gently layering trichloroacetic acid (50% TCA, 50 μl) on top of the medium in all the wells. The plates were incubated at 4°C for one hour to fix the cells attached to the bottom of the wells. The liquid of all the wells was gently pipetted out and discarded. The plates were washed five times with distilled water to remove TCA, growth medium low molecular weight metabolites, serum proteins etc. and air - dried. Cell growth was measured by staining with sulforhodamine B dye. The adsorbed dye was dissolved in Tris-HCl Buffer (100 μl/well, 0.01M, pH 10.4) and plates were gently stirred for 10 minutes on a mechanical stirrer. The optical density (OD) was recorded on ELISA reader at 540nm. Paclitaxel (Taxol), and 5-Fluorouracil were used as positive controls (Standard drugs).

**C. *In-vitro* tubulin polymerisation inhibition assay**

The tubulin polymerization experiment was performed using 'assay kit'(Cytoskeleton USA) following manufacturer's protocol based on the procedure described by of Shelanski et al, (1973) and Lee et al. (1977). Briefly, tubulin protein (3mg/mL) in tubulin polymerization buffer (80 mM PIPES, pH 6.9, 2 mM MgCl2, 0.5 mM EGTA, 1 mM GTP and 15% glycerol) was placed in prewarmed 96-well microtiter plates at 37° C in the presence of test compounds with variable concentrations. All samples were mixed well and polymerization was monitored kinetically at 340 nm every min for 1h using a Spectramax plate reader. The $IC_{50}$ value was determined from dose-dependent analysis and is defined as the concentration that inhibits the rate of polymerization by 50%. Podophyllotoxin was used as standard tubulin polymerisation inhibitor as positive control.

**Table 3:** ***In-vitro*** **anticancer (cytotoxicity) activity and tubulin polymerisation inhibition of 2-benzyl-indanones (2-5)**

| | **Human cancer cell lines $IC_{50}$ (μM)*** | | | | | |
|---|---|---|---|---|---|---|
| **Compd. no.** | **A549 (Lung)** | **THP-1 (Leukemia)** | **HCT-116 (Colon)** | **MCF-7 (Breast cancer)** | **T-47D (Breast cancer)** | **Tubulin polymerisation inhibition $IC_{50}$ (μM)** |
| **2** | 2.0 | 1.0 | 3.0 | 3.0 | <1.0 | 2.08 |
| **4** | 24 | 55 | 22 | 45 | 36 | -- |
| **5** | 1.0 | 1.0 | 4.0 | 12.0 | 12.0 | -- |
| **12 (parent compound)** | 11.0 | 4.9 | 0.10 | 0.01 | --- | 0.63 |
| **Podophyllotoxin** | --- | --- | --- | --- | --- | 0.31 |

| **Positive controls** | | **% Inhibition** | | | | | |
|---|---|---|---|---|---|---|---|
| | **Concentration (μM)** | **HCT (Colon)** | **Hela (Cervix)** | **THP-1 (Leukemia)** | **A549 (Lung)** | **T-47D (Breast)** | **MCF-7 (Breast)** |
| 5-Fluorouracil | 20 | 88 | 70 | 90 | 54 | 60 | 50 |
| Paclitaxel | 1 | 45 | - | 60 | 88 | 78 | 77 |

From table 3, it is evident that compound **12** possesses better cytotoxic activity than any other optimized 2-benzylindanone (**2, 4, 5**) against MCF-7 and HCT-116. However, compound **2,** possessed better activity against A549 and THP-1 cell lines than compound **12.** Overall, compound **2** possessed significant anticancer activity against various human cancer cell lines. Compounds 4, and **5** exhibited comparatively low activity.

C. ***In vivo*** **anticancer activity of compound 2 against Ehrlich Ascites Carcinoma (EAC)**

| | |
|---|---|
| Model: | Ehrlich Ascites Carcinoma (EAC) |
| Animals: | Swiss mice |
| Sex: | Female |
| Weight: | 18-23 g |

Ehrlich ascites carcinoma (EAC) cells were collected from the peritoneal cavity of the Swiss mice harbouring 8-10 days old ascitic tumor. $1 \times 10^7$ EAC cells were injected intraperitoneally in Swiss mice selected for the experiment on day 0. The next day, animals were randomized and divided into different groups. The treatment groups contained 7 animals each and control group contained 10 animals. The treatment group was treated with 20 mg/kg of Compound **2** intra-peritoneally from day 1-9. One more of the treatment group received 5-fluorouracil (20 mg/kg, i.p) and it served as positive control. The tumor bearing control group was similarly administered normal saline (0.2 ml, i.p.). On day 12, animals were sacrificed and ascitic fluid was collected from peritoneal cavity of each mouse for the evaluation of tumor growth. Percent tumor growth inhibition was calculated based on the total number of tumor cells present in the peritoneal cavity as on day 12 of the experiment using the following formula.

$$\text{Percent tumor growth inhibition} = \frac{\text{Av. no. of cells in control group} - \text{Av. no. of cells in treated group}}{\text{Av. no. of cells in control group}} \times 100$$

The results are presented in tables 2 & 3.

**Table 4.** Body weight of animals during the experiment

| Treatment | Dose | Body weight (g) | | | |
|---|---|---|---|---|---|
| | | Day 1 | Day 5 | Day 9 | Day 12 |
| Control | 0.2 ml N.S. i/p | 21.14 | 22.71 | 22.71 | 22.14 |
| **2** | 20 mg/kg i/p | 21.0 | 21.86 | 22.28 | 22.66 |
| **12** (Parent compound) | 20 mg/kg i/p | 21.42 | 22.85 | 22.42 | 23.16 |
| 5-Fluorouracil | 20 mg/Kg i/p | 21.9 | 23.1 | 24.5 | 25.6 |

**Table 5** Antitumour activity of 2-benzylindanone compounds **2.**

| Treatment | Dose | Day 12 | | | | | |
|---|---|---|---|---|---|---|---|
| | | Av, Volume of ascitic fluid (ml) | Av. Weight of ascitic fluid (g) | Av. No. of tumor cells | % Tumor cell growth | % Tumor growth inhibition | Mortality |
| Control | 0.2 ml N.S. i/p | 9.96 | 10.33 | $138.42 \times 10^7$ | 100 | - | 0/10 |
| **2** | 20 mg/kg i/p | 6.31 | 6.02 | $44.78 \times 10^7$ | 32.36 | **67.64** | 0/7 |

(continued)

| Treatment | Dose | Day 12 | | | | | |
|---|---|---|---|---|---|---|---|
| | | Av, Volume of ascitic fluid (ml) | Av. Weight of ascitic fluid (g) | Av. No. of tumor cells | % Tumor cell growth | % Tumor growth inhibition | Mortality |
| **12** (Parent compound | 20 mg/kg i/p | 6.11 | 5.88 | 75.46 | 54.52 | **45.48** | 0/7 |
| 5-Fluorouracil | 20 mg/Kg i/p | 0.7 | 0.46 | $6.33 \times 10^7$ | 4.6 | 95.4 | 0/7 |

2-Benzylindanone **2** exhibited potent *in-vivo* anticancer activity by reducing Ehrlich ascite carcinoma by 67.64% in Swiss-albino mice at 20mg/kg dose. While, the parent lead **12** induced 45.5% of tumour reduction at 20mg/kg dose in Ehrlich ascites carcinoma. Thus, compound **2** has 48.72% better activity *(in-vivo)* over the parent compound **12.**

**D. Anticancer Activity of compound 2 against Ehrlich Tumor (Solid)**

| | |
|---|---|
| **Model:** | Ehrlich Tumor (solid) |
| **Samples:** | **2** (20 mg/kg i/p) |
| **Animals:** | Swiss |
| **Sex:** | Males |
| **Weight:** | 18-23 g |

Ehrlich ascites carcinoma (EAC) cells were collected from the peritoneal cavity of the Swiss mice harbouring 8-10 days old ascitic tumor. $1 \times 10^7$ EAC cells were injected intramuscularly in right thigh of Swiss male mice selected for the experiment on day 0. The next day, animals were randomized and divided into different groups. Two treatment groups contained 7 animals each and one control group contained 10 animals. The first treatment group was given compound 2 (20 mg/kg i/p) from day 1-9. The second treatment group was treated with 5-fluorouracil (22 mg/kg, i.p) from day 1-9 and it served as positive control. The control group was similarly administered normal saline (0.2 ml, i.p.) from day 1-9. On day 9 & 13, tumor bearing thigh of each animal was shaved and longest and shortest diameters of the tumor were measured with the help of vernier caliper. Tumor weight of each animal was calculated using the following formula.

$$\text{Tumor weight (mg)} = \frac{\text{Length (mm)} \times [\text{width(mm)}]^2}{2}$$

The percent tumor growth inhibition was calculated on day 13 by comparing the average values of treated groups with that of control group. Tumor growth in saline treated control animals was taken to be 100%. The results are presented in table 6.

**Table 6.** In-vivo anticancer activity of 2 against Ehrlich ascites carcinoma (solid)

| **Treatment Groups** | **Av. Body weights (g) of animals on days** | | | **Day 13** | | **%Tumor Growth** | **Mortality** |
|---|---|---|---|---|---|---|---|
| | **1** | **5** | **9** | **Av. Body weights (g)** | **Av. Tumor weights (mg)** | **Inhibition** | |
| **2 (20 mg/kg i/p)** | **21.42** | **23.14** | **23.42** | **23.28** | **1178.35±32.72** | **42.77** | **0/7** |
| **12 (20 mg/kg i/p)** | 21.42 | 22.85 | 22.42 | 23.71 | 1319.64±56.46 | **35.91** | 0/7 |
| Positive control 5 FU (22 mg /Kg i/p) | 21.71 | 21.42 | 20.71 | 21.0 | 842.85±129.92 | 59.07 | 0/7 |
| Normal Control Normal saline (NS) (0.2 ml i/p) | 22.8 | 24.3 | 24.4 | 25.5 | 2059.25±102.163 | 0 | 0/10 |

Compound **2** reduced solid tumour by 42.771% at 20mg/kg dose, while the parent lead **12** reduced solid tumour by 35.9% at 20mg/kg dose in Ehrlich ascites carcinoma. Thus, in case of solid tumour compound **2** has 19.1% better activity (*in-vivo*) over the parent compound **12** at 20mg/kg dose.

**E. *In-vivo* acute oral toxicity in Swiss-albino mice**

In view of potent anti-cancer activity of compound **2** in *in-vitro* model, acute and sub-acute oral toxicity of the same was carried out in Swiss albino mice for its further development into drug product. Experiment was conducted in accordance with the Organization for Economic Cooperation and Development (OECD) test guideline No 423 (1987).

For the acute oral toxicity study, 30 mice (15 male and 15 female) were taken and divided into four groups comprising 3 male and 3 female mice in each group weighing between 20-25 g. The animals were maintained at 22±5°C with humidity control and also on an automatic dark and light cycle of 12 hours. The animals were fed with the standard mice feed and provided *ad libitum* drinking water. Mice of group 1 (Group 1) were kept as control and animals of groups 2, 3, 4 and 5 (Groups II, III, IV & V) were kept as experimental. The animals were acclimatized for 7 days in the experimental environment prior to the actual experimentation. The test compound **2** was solubilized in dimethyl sulphoxide and then suspended in double distilled water (DDW) and was given at 5, 50, 300 and 1000mg/kg body weight to animals of groups 2, 3, 4 and 5 (Groups II, III, IV & V) respectively once orally. Control animals received only vehicle.

***Observational, haematological, biochemical and gross pathological study***

The animals were checked for mortality and any signs of ill health at hourly interval on the day of administration of drug and there after a daily general case side clinical examination was carried out including changes in skin, mucous membrane, eyes, occurrence of secretion and excretion and also responses like lachrymation, piloerection respiratory patterns etc. Also changes in gait, posture and response to handling were also recorded. In addition to observational study, body weights were recorded and blood and serum samples were collected from all the animals on $7^{th}$ day of the experiment in acute oral toxicity. The samples were analysed for total RBC, WBC, differential leucocytes count, haemoglobin percentage and biochemical parameters like ALP, SGPT, SGOT, total cholesterol, triglycerides, creatinine, bilirubin, serum protein, tissue protein, malonaldehyde and reduced GSH activity. The animals were then sacrificed and were necropsed for any gross pathological changes. Weights of vital organs like liver, heart, kidney etc. were recorded.

**Table 7.** Effect of compound **2** as a single acute oral dose at 5, 50, 300 and 1000 mg/kg on body weight, haematological and serum biochemical parameters in Swiss albino mice (Mean±SE; n=6; a, P<0.001 compared to control, **5, 50, 300, 1000** mg/kg)

| **Parameters** | **Dose of 2 at mg/kg body weight as a single oral dose** | | | | |
|---|---|---|---|---|---|
| | **Control** | **5 mg/kg** | **50 mg/kg** | **300 mg/kg** | **1000 mg/kg** |
| **Body weight (gm)** | 24.03±0.62 | 23.97±0.64 | 24.29±0.95 | 23.83±0.42 | 23.41±0.16 |

(continued)

| Parameters | Dose of 2 at mg/kg body weight as a single oral dose | | | | |
|---|---|---|---|---|---|
| | Control | 5 mg/kg | 50 mg/kg | 300 mg/kg | 1000 mg/kg |
| **Haemoglobin (gm/dL)** | 11.04±0.65 | 13.55±0.60 | 13.06±0.82 | 10.90±1.11 | 12.29±0.53 |
| **RBC (million/mm3)** | 6.18±0.20 | 5.93±0.39 | 5.83±0.17 | 6.03±0.11 | 6.10±0.27 |
| **WBC (1000*/mm3)** | 7.83±0.70 | 7.95±0.64 | 7.99±0.55 | 8.13±0.64 | 8.57±0.76 |
| **ALP (U/L)** | 264.1±20.6 | 167.3±16.5 | 191.6±27.6 | 224.0±6.70 | 234.3±44.5** |
| **SGOT (U/L)** | 31.49±6.17 | 12.24±1.18 | 25.76±5.40 | 18.71±3.16 | 35.49±6.57 |
| **SGPT (U/L)** | 16.99±1.71 | 15.74±1.03 | 17.92±1.69 | 18.20±1.41 | 11.97±1.67 |
| **Albumin (g/dL)** | 3.46±0.09 | 3.63±0.28 | 3.41±0.0.33 | 3.63±0.28 | 3.26±0.30 |
| **Creatinine (mg/dL)** | 0.47±0.06 | 0.56±0.10 | 0.69±0.08 | 0.76±0.03 | 0.76±0.09 |
| **Triglycerides (mg/dL)** | 91.82±5.66 | 98.74±3.11 | 74.95±5.79 | 104.94±10.39 | 87.28±4.39 |
| **Serum Protein (mg/ml)** | 1.13±0.09 | 1.07±0.06 | 1.13±0.07 | 1.09±0.06 | 1.42±0.16 |
| **Cholesterol (mg/dL)** | 107.7±5.4 | 104.0±6.7 | 120.3±10.4 | 116.5±15.6 | 115,5±8.8 |
| **Bilirubin (mg/dL)** | 0.06±0.01 | 0.14±0.07 | 0.09±0.01 | 0.08±0.D2 | 0.12±0.04 |

No observational changes, morbidity and mortality were observed throughout the experimental period up to the dose level of 1000 mg/kg body weight. No morbidity or any other gross observation changes could be noticed in the group of animals treated with the test drug (compound **2**) at 1000 mg/kg. Blood and serum samples upon analysis showed non-significant changes in all the parameters studied like total haemoglobin level, RBC count, WBC count, differential leucocytes count, SGPT, ALP, creatinine, triglycerides, cholesterol, albumin, serum protein (Table 7 & Fig. 2). However, ALP showed significant changes in the animals treated with the test drug at 1000 mg/kg body weight as compare to control groups. Animals on gross pathological study showed no changes in any of the organs studied including their absolute and relative weight (Figure 1). Therefore, the experiment showed that compound **2** is well tolerated by the Swiss albino mice up to the dose level of 1000 mg/kg body weight as a single acute oral dose. However, sub-acute and or chronic experiment with the test drug needs to be carried out to look for any adverse effect on repeated exposure to the test drug compound **2** (AS6H) for its future development.

Overall, the present compound 2-benzyl-indanones (**1**) showed potent anticancer activity mainly against Leukemia (THP-1), lung (A549), colon (HCT-116) and breast cancer cell lines (MCF-7, T47D). It exhibited inhibition of tubulin polymerase. Microtubules play an essential role in cell division. Tubulin polymerisation inhibitors inhibit mitosis or cell division. Such a molecule prevents cancer cells to undergo mitosis by disrupting microtubule polymerisation and hence used in cancer treatment.

## ADVANTAGES OF THE PRESENT INVENTION

This is a novel compound exhibiting in vitro and in-vivo anticancer (cytotoxic) activity through tubulin polymerisation inhibition and non-toxic. This is also the first process of preparation of this compound. It can be prepared from simple starting compound. Reaction procedures are also simple and straight forward. Hence, it will be very economic also.

## Claims

1. A compound of general formula 1

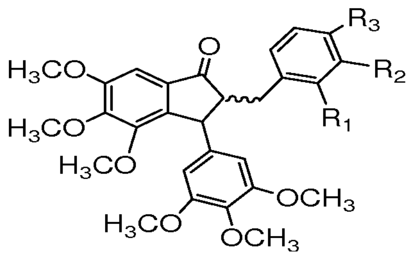

1

wherein $R_1$, $R_2$, and $R_3$ are selected from a group consisting of H, Halogen, alkyl, alkoxy, alkylenedioxy, hydroxy, and aryl;
wherein, $R_3$ is not hydroxy when $R_1$ and $R_2$ are H.

**2.** The compound as claimed in claim 1, wherein the structural formula of the compound comprising;

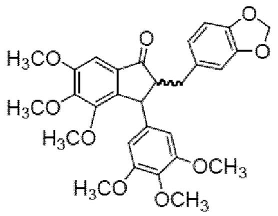

2

2-(3',4'-methylenedioxybenzyl)-3-(3",4",5"-trimethoxyphenyl)-indanone-1;

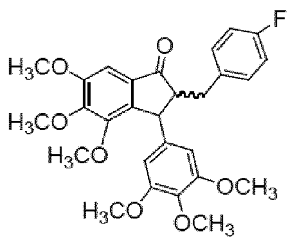

4

2-(4'-fluorobenzyl)-3-(3",4",5"-trimethoxyphenyl)-indanone-1;

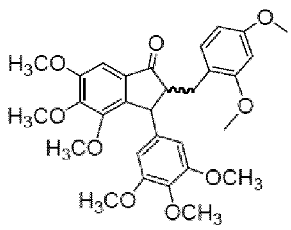

5

2-(2',4'-dimethoxybenzyl)-3-(3",4",5 "-trimethoxyphenyl)-indanone-1.

**3.** The compound as claimed in claim 1, wherein the compound represented by formula **2** shows tubulin polymerisation inhibition activity.

**4.** The compound as claimed in claim 1, wherein the compound is useful as an anticancer agent.

**5.** The compound as claimed in claim 1, wherein the compound represented by formula **2** exhibits cytotoxicity against various human cancer cell lines, $IC_{50}$ ranging from 1μM to 3 μM.

**6.** The compound as claimed in claim 5, wherein the compound shows no toxicity in *in-vivo* acute oral toxicity up to 300mg/kg.

**7.** The compound as claimed in claim 6, wherein the compound exhibits *in-vivo* anticancer activity in Swiss-albino mice reduces Ehrlich ascite carcinoma by 67.64% and solid tumour by 42.77% at 20 mg/kg dose.

**8.** A process for the preparation of the compounds of general formula **1** as claimed in claim 1, wherein said process comprises the steps of:

a. adding a compound selected from a group consisting of 12, 14 and 15,

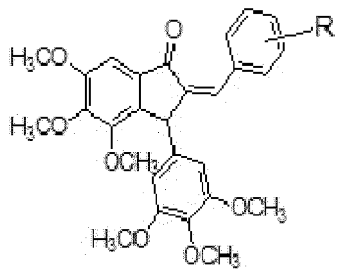

**12**: R= 3,4,-methylenedioxy;
**14**: 4-Fluoro; **15**: 2,4-Dimethoxy

in a solvent under stirring at a temperature ranging between 20-45°C for a period of time ranging between 5-10 min to get solution;
b. adding catalyst to the solution obtained from step (a) under hydrogen atmosphere followed by stirring at a temperature ranging between 20-45°C for a period of time ranging between 2-5 h to get reaction mixture;
c. filtering the reaction mixture obtained from step (b) followed by washing and drying to get pure product.

**9.** The process as claimed in claim 8, wherein the solvent used in step (a) is selected from the group consisting of methanol, dioxane, ethyl acetate and tetrahydrofuran.

**10.** The process as claimed in claim 8, wherein the catalyst used in step (b) is selected from the group consisting of Raney-Ni, RhCl (PPh3) and Pd-C.

**11.** The process as claimed in claim 8, wherein the amount of catalyst used in step (b) is in the range of 0.5 g to 1.0g per gram of substrate.

**12.** The process as claimed in claim 8, wherein the yield of the compound **1** of general formula 1 is in the range of 40-83%.

**13.** A pharmaceutical composition for the treatment of cancer wherein the composition comprises an effective amount of the compound of general formula 1 as claimed in claim 1; optionally, along with the pharmaceutically acceptable excipients, diluents, additives.

**14.** A compound of general formula 1 for use in a method of treatment of cancer or any other disease related to tubulin disorders, wherein the method comprises administering in a therapeutically effective amount of one or more compounds of formula 1;

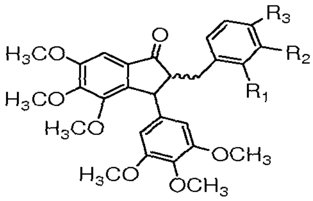

1

Where $R_1$, $R_2$ and $R_3$ are selected from a group consisting of H, halogen, alkyl, alkoxy, akylenedioxy, hydroxy, and aryl;
wherein, $R_3$ is not hydroxy when $R_1$ and $R_2$ are H.

## Patentansprüche

1. Verbindung der allgemeinen Formel 1

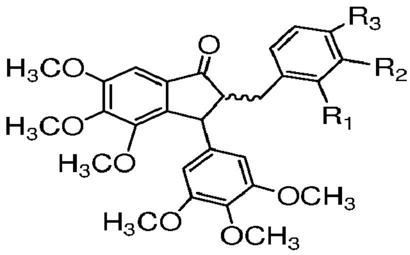

1

wobei $R_1$, $R_2$ und $R_3$ ausgewählt sind aus der Gruppe bestehend aus H, Halogen, Alkyl, Alkoxy, Akyldioxy, Hydroxy und Aryl;
wobei $R_3$ nicht für Hydroxy steht, wenn $R_1$ und $R_2$ für H stehen.

2. Verbindung nach Anspruch 1, wobei die Strukturformel der Verbindung

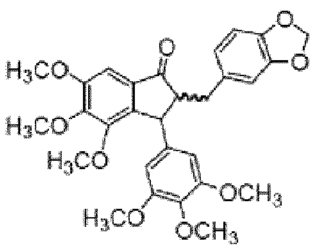

2

2-(3',4'-Methylendioxybenzyl)-3-(3",4", 5"-trimethoxhenl-indanon-1;

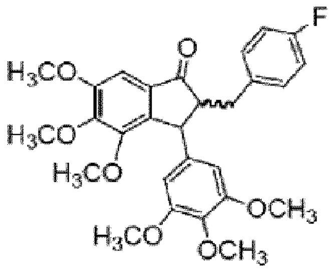

**4**

2-(4'-Fluorbenzyl)-3-(3",4",5"-trimethoxyphenyl)-indanon-1 ;

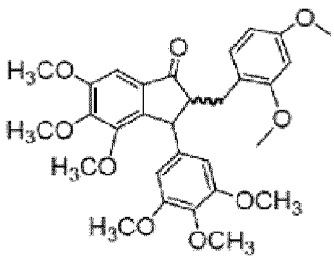

**5**

2-(2',4'-Dimethoxybenzyl)-3-(3",4",5"-trimethoxyphenyl)-indanon-1 umfasst.

3. Verbindung nach Anspruch 1, wobei die durch Formel **2** dargestellte Verbindung eine Aktivität zur Hemmung der Tubulinpolymerisation zeigt.

4. Verbindung nach Anspruch 1, wobei die Verbindung als ein Antikrebsmittel nützlich ist.

5. Verbindung nach Anspruch 1, wobei die durch Formel **2** dargestellte Verbindung eine Zytotoxizität gegenüber verschiedenen humanen Krebszelllinien aufweist, wobei der $IC_{50}$-Wert im Bereich von 1 $\mu$M bis 3 $\mu$M liegt.

6. Verbindung nach Anspruch 5, wobei die Verbindung bei akuter oraler Toxizität in vivo bis zu 300 mg/kg keine Toxizität zeigt.

7. Verbindung nach Anspruch 6, wobei die Verbindung in vivo eine Antikrebsaktivität bei Swiss-Albino-Mäusen zeigt, die Ehrlich-Ascites-Karzinom um 67,64 % und festen Tumor um 42,77 % bei einer Dosis von 20 mg/kg reduziert.

8. Verfahren zur Herstellung der Verbindungen der allgemeinen Formel **1** nach Anspruch 1, wobei das Verfahren die folgenden Schritte umfasst:

   a. Zugeben einer Verbindung, ausgewählt aus einer Gruppe bestehend aus 12, 14 und 15,

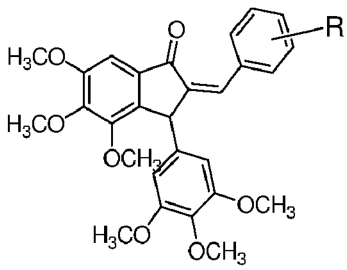

**12**: R = 3,4,-Methylendioxy;

**14**: 4-Fluor; **15**: 2,4-Dimethoxy

zu einem Lösungsmittel unter Rühren bei einer Temperatur im Bereich von 20-45 °C für einen Zeitraum im Bereich von 5-10 Min., um eine Lösung zu erhalten;

b. Zugeben eines Katalysators zu der aus Schritt (a) erhaltenen Lösung unter Wasserstoffatmosphäre, gefolgt von Rühren bei einer Temperatur im Bereich von 20-45 °C für einen Zeitraum im Bereich von 2-5 Std., um das Reaktionsgemisch zu erhalten;

c. Filtrieren des aus Schritt (b) erhaltenen Reaktionsgemischs, gefolgt von Waschen und Trocknen, um ein reines Produkt zu erhalten.

**9.** Verfahren nach Anspruch 8, wobei das in Schritt (a) verwendete Lösungsmittel ausgewählt ist aus der Gruppe bestehend aus Methanol, Dioxan, Ethylacetat und Tetrahydrofuran.

**10.** Verfahren nach Anspruch 8, wobei der in Schritt (b) verwendete Katalysator ausgewählt ist aus der Gruppe bestehend aus Raney-Ni, RhCl (PPh3) und Pd-C.

**11.** Verfahren nach Anspruch 8, wobei die in Schritt (b) verwendete Katalysatormenge im Bereich von 0,5 g bis 1,0 g pro Gramm Substrat liegt.

**12.** Verfahren nach Anspruch 8, wobei die Ausbeute der Verbindung 1 der allgemeinen Formel 1 im Bereich von 40-83 % liegt.

**13.** Pharmazeutische Zusammensetzung zur Behandlung von Krebs, wobei die Zusammensetzung eine wirksame Menge der Verbindung der allgemeinen Formel 1 nach Anspruch 1; gegebenenfalls zusammen mit pharmazeutisch verträglichen Hilfsstoffen, Verdünnungsmitteln, Additiven, umfasst.

**14.** Verbindung der allgemeinen Formel 1 zur Verwendung in einem Verfahren zur Behandlung von Krebs oder jeder anderen Erkrankung, die mit Tubulinstörungen zusammenhängt, wobei das Verfahren das Verabreichen in einer therapeutisch wirksamen Menge einer oder mehrerer Verbindungen der Formel 1 umfasst;

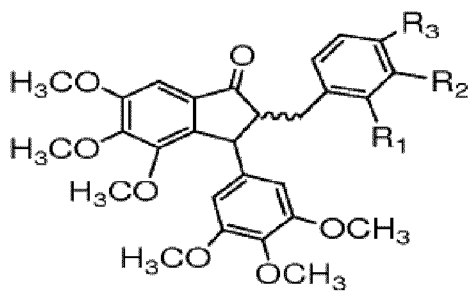

**1**

wobei $R_1$, $R_2$ und $R_3$ ausgewählt sind aus der Gruppe bestehend aus H, Halogen, Alkyl, Alkoxy, Akyldioxy, Hydroxy und Aryl;

wobei $R_3$ nicht für Hydroxy steht, wenn $R_1$ und $R_2$ für H stehen.

## Revendications

**1.** Composé de formule générale 1

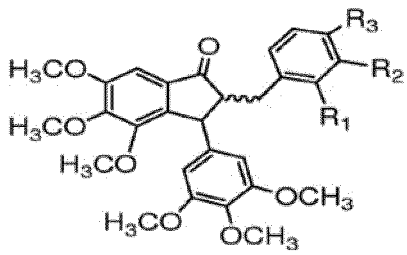

1

dans laquelle $R_1$, $R_2$ et $R_3$ sont sélectionnés dans un groupe constitué par H, un halogène, un alkyle, un alcoxy, un alkylènedioxy, un hydroxy et un aryle ; dans laquelle $R_3$ n'est pas un hydroxy lorsque $R_1$ et $R_2$ représentent H.

**2.** Composé selon la revendication 1, dans lequel la formule développée du composé comprenant ;

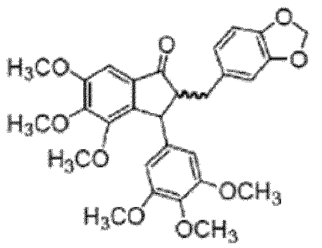

2

2-(3',4'-méthylènedioxybenzyl)-3-(3",4",5"-triméthoxyphényl)-indanone-1 ;

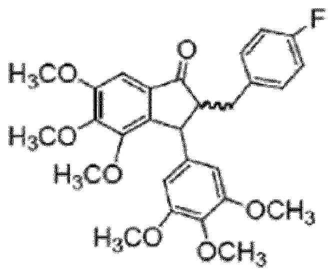

4

2-(4'-fluorobenzyl)-3-(3",4",5"-triméthoxyphényl)-indanone-1 ;

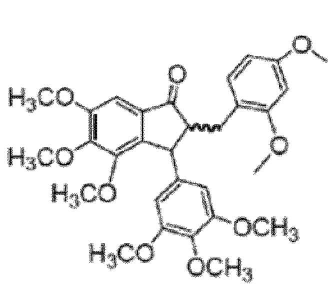

5

2-(2',4'-diméthoxybenzyl)-3-(3",4",5"-triméthoxyphényl)-indanone-1.

**3.** Composé selon la revendication 1, dans lequel le composé représenté par la formule 2 montre une activité d'inhibition de la polymérisation de la tubuline.

**4.** Composé selon la revendication 1, dans lequel le composé est utile en tant qu'agent anticancéreux.

**5.** Composé selon la revendication 1, dans lequel le composé représenté par la formule 2 présente une cytotoxicité contre les lignées cellulaires de divers cancers humains, $CI_{50}$ comprise dans la plage allant de 1 $\mu$M à 3 $\mu$M.

**6.** Composé selon la revendication 5, dans lequel le composé montre aucune toxicité dans une toxicité orale aiguë *in-vivo* jusqu'à 300 mg/kg.

**7.** Composé selon la revendication 6, dans lequel le composé présente une activité anticancéreuse *in-vivo* chez des souris Swiss-albino qui réduit le carcinome d'Ehrlich ascite de 67,64 % et une tumeur solide de 42,77 % à une dose de 20 mg/kg.

**8.** Procédé de préparation des composés de formule générale 1 selon la revendication 1, dans lequel ledit procédé comprend les étapes consistant à :

a. ajouter un composé sélectionné dans un groupe constitué par 12, 14 et 15,

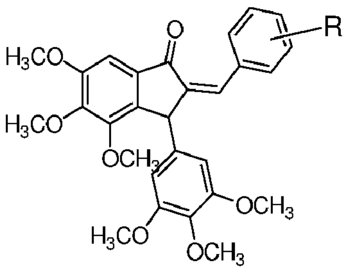

12 : R = 3,4-méthylènedioxy ;

14 : 4-fluoro ; 15 : 2,4-diméthoxy

dans un solvant sous agitation à une température comprise entre 20 et 45 °C pendant une période de temps comprise entre 5 et 10 min pour obtenir une solution ;

b. ajouter un catalyseur à la solution obtenue à l'étape (a) sous une atmosphère d'hydrogène puis agiter à une température comprise entre 20 et 45 °C pendant une période de temps comprise entre 2 et 5 h pour obtenir un mélange réactionnel ;

c. filtrer le mélange réactionnel obtenu à l'étape (b) puis laver et sécher pour obtenir le produit pur.

**9.** Procédé selon la revendication 8, dans lequel le solvant utilisé à l'étape (a) est sélectionné dans le groupe constitué par le méthanol, le dioxane, l'acétate d'éthyle et le tétrahydrofurane.

**10.** Procédé selon la revendication 8, dans lequel le catalyseur utilisé à l'étape (b) est sélectionné dans le groupe constitué par le Ni de Raney, $RhCl(PPh_3)$ et Pd-C.

**11.** Procédé selon la revendication 8, dans lequel la quantité de catalyseur utilisé à l'étape (b) est comprise dans la plage allant de 0,5 g à 1,0 g par gramme de substrat.

**12.** Procédé selon la revendication 8, dans lequel le rendement du composé 1 de formule générale 1 est compris dans la plage allant de 40 à 83%.

**13.** Composition pharmaceutique pour le traitement d'un cancer dans laquelle la composition comprend une quantité efficace du composé de formule générale 1 selon la revendication 1 ; éventuellement avec des excipients, diluants, additifs pharmaceutiquement acceptables.

**14.** Composé de formule générale 1 pour une utilisation dans une méthode de traitement d'un cancer ou de n'importe

quelle autre maladie liée aux troubles de la tubuline, dans lequel la méthode comprend l'administration selon une quantité thérapeutiquement efficace d'un ou plusieurs composés de formule 1 ;

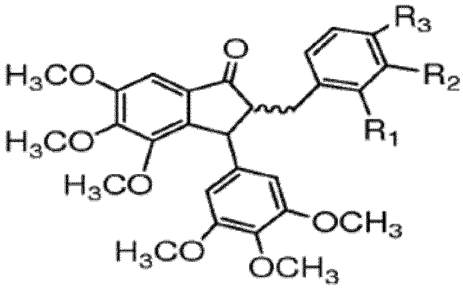

**1**

dans laquelle $R_1$, $R_2$ et $R_3$ sont sélectionnés dans un groupe constitué par H, un halogène, un alkyle, un alcoxy, un alkylènedioxy, un hydroxy et un aryle ; dans laquelle $R_3$ n'est pas un hydroxy lorsque $R_1$ et $R_2$ représentent H.

Figure 1.

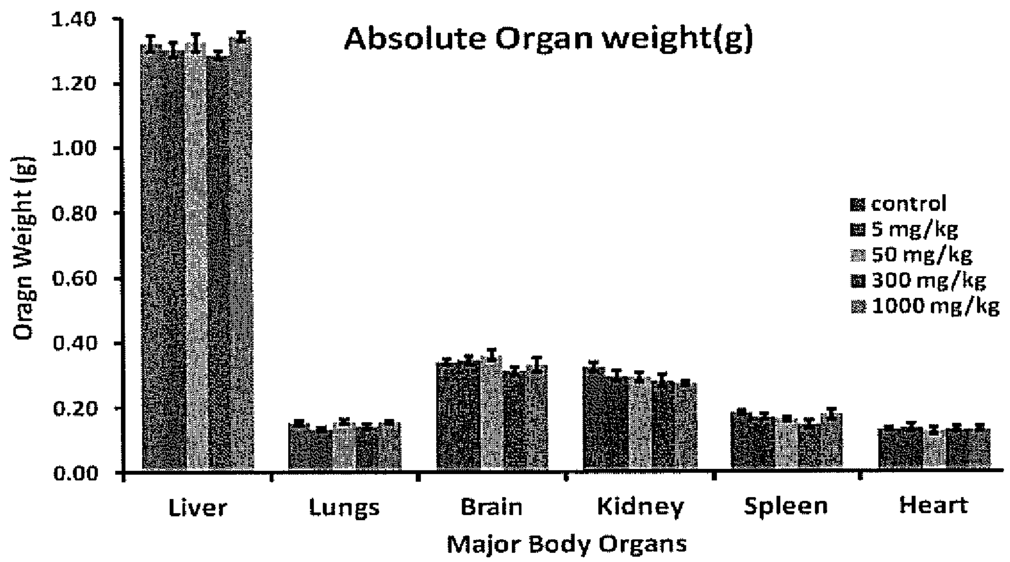
Absolute Organ weight(g)
1.40
1.20
1.00
0.80
0.60
0.40
0.20
0.00
Oragn Weight (g)
control
5 mg/kg
50 mg/kg
300 mg/kg
1000 mg/kg
Liver
Lungs
Brain
Kidney
Spleen
Heart
Major Body Organs

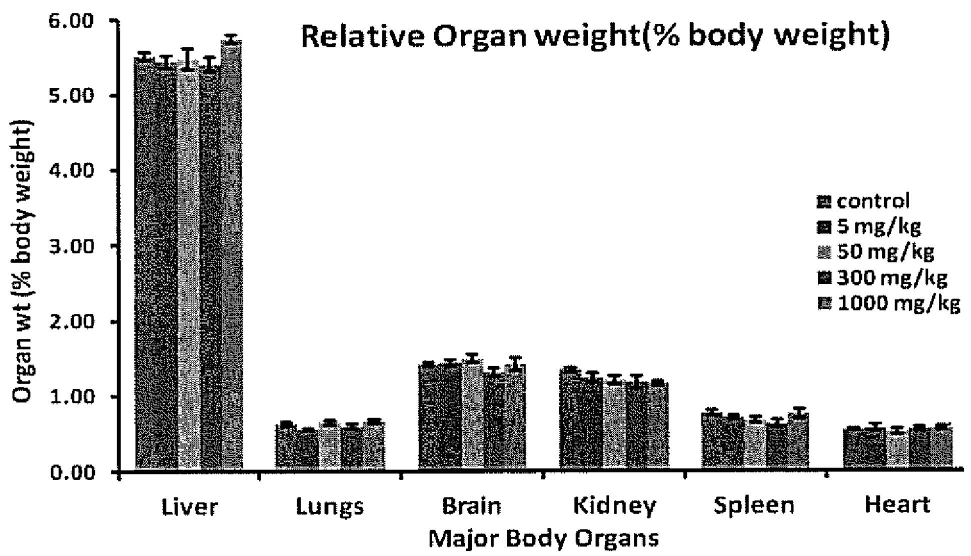
Relative Organ weight(% body weight)
6.00
5.00
4.00
3.00
2.00
1.00
0.00
Organ wt (% body weight)
control
5 mg/kg
50 mg/kg
300 mg/kg
1000 mg/kg
Liver
Lungs
Brain
Kidney
Spleen
Heart
Major Body Organs

Figure 2.

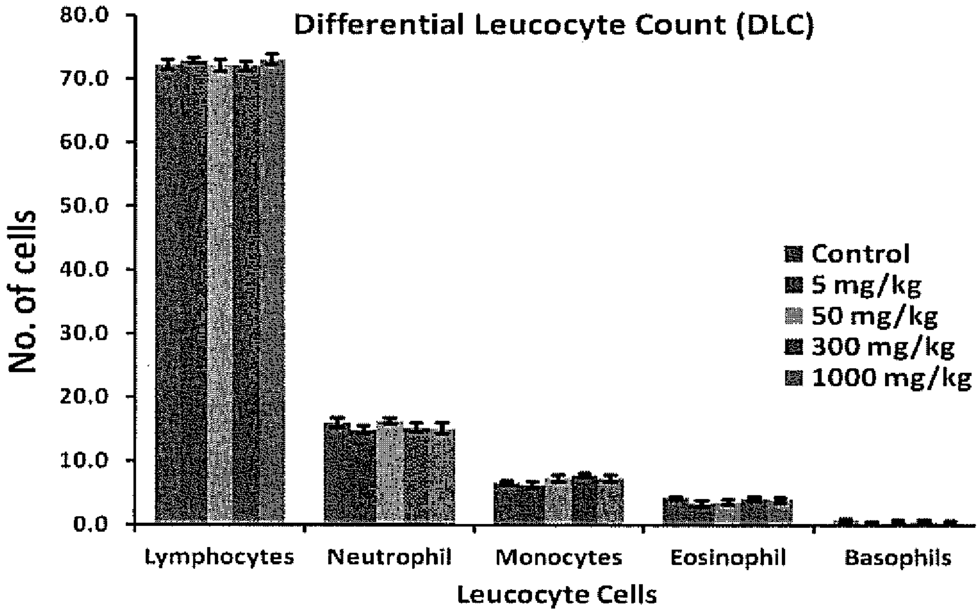
Differential Leucocyte Count (DLC)
80.0
70.0
60.0
50.0
40.0
30.0
20.0
10.0
0.0
No. of cells
Control
5 mg/kg
50 mg/kg
300 mg/kg
1000 mg/kg
Lymphocytes
Neutrophil
Monocytes
Eosinophil
Basophils
Leucocyte Cells

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- JP 08198798 A
- HU 9903620 A2
- WO 2007031833A2 A, Bansal
- US 8633242 B2, Negi


### Non-patent literature cited in the description


- **LOCATELLI et al.** *Eur. J. Med. Chem.,* 2013, vol. 60, 233-239
- **PETTIT et al.** *J. Nat. Prod.,* 2000, vol. 63 (7), 969-974
- **SYRCHINA ; SEMENOV.** *Chem, Nat. Compds.,* 1982, vol. 18, 1-11
- **LAWRENCE et al.** *Tetrahedron Lett.,* 2006, vol. 47, 1637
- **LEONI et al.** *J. Natl. Cancer Inst.,* 2000, vol. 92, 217
- *Biochem. Pharmacol.,* 2012, vol. 83, 1495-506
- **DAS et al.** *Biochemistry,* 2009, vol. 48, 1628-1635
- **BRENDEL K.** *Hung. Pat. Appl.,* 2000
- **PRAKASHAM.** *Biorganic and Medicinal Chemistry,* 2012, vol. 20 (9), 3049-3057
- **SKEHAN et al.** *J. Natl. Cancer Inst.,* 1990, vol. 82, 1107